**Europäisches Patentamt**

**European Patent Office**

**Office européen des brevets**

(19)

(11) Veröffentlichungsnummer: **0 120 994**

**A1**

# EUROPÄISCHE PATENTANMELDUNG

(12)

(21) Anmeldenummer: **83112289.0**

(22) Anmeldetag: **07.12.83**

(51) Int. Cl.³: **C 07 C 109/087**

(30) Priorität: **07.01.83 DE 3300314**

(43) Veröffentlichungstag der Anmeldung:
**10.10.84 Patentblatt 84/41**

(84) Benannte Vertragsstaaten:
**AT BE CH DE FR GB IT LI LU NL SE**

(71) Anmelder: **DYNAMIT NOBEL AKTIENGESELLSCHAFT**
**Postfach 1209**
**D-5210 Troisdorf, Bez. Köln(DE)**

(72) Erfinder: **Burkhardt, Rudolf, Dr.**
**Im Hahn 1**
**D-5461 Vettelschoss(DE)**

(72) Erfinder: **Theis, Christoph, Dr.**
**Hoher Rain 5**
**D-5216 Niederkassel 6(DE)**

(54) **Verfahren zur Herstellung von N,N'-Diformylhydrazin.**

(57) Es wird ein Verfahren zur Herstellung von N,N'-Di-formyl-hydrazin aus Hydrazinhydrat bzw. ggf. Monoformylhydrazin und Ameisensäure oder Formamid durch Umsetzung in einem inerten Lösungsmittel und anschließender Destillation eines azeotropen Gemischs aus dem jeweiligen inerten Lösungsmittel und Wasser vorgeschlagen.

EP 0 120 994 A1

Croydon Printing Company Ltd.

Troisdorf, 10. Dezember 1982
OZ 83002   (4211)  Dr. La/cp

DYNAMIT NOBEL AKTIENGESELLSCHAFT
Troisdorf, Bez. Köln

Verfahren zur Herstellung von N,N'-Diformylhydrazin

Die Erfindung betrifft ein Verfahren zur Herstellung von N,N'-Diformylhydrazin der Formel OCH-NH-NH-CHO aus Hydrazinhydrat bzw. ggf. Monoformylhydrazin und Ameisensäure oder Formamid.

Es ist bekannt, N,N'-Diformylhydrazin aus Hydrazinhydrat und Ameisensäure oder Formamid nach C. Ainsworth und R.G. Jones, J. Am. Chem. Soc. 77, 621-4 (1955) herzustellen, jedoch werden trotz langer Reaktionszeiten nur Ausbeuten von etwa 80 %, bezogen auf Hydrazinhydrat, erhalten. Die Herstellung aus wasserfreiem Hydrazin ist möglich, erfordert aber wesentlich erhöhte Sicherheitsvorkehrungen. Bei Verwendung von Hydrazinhydrat kann die Ausbeute bis über 95 % gesteigert werden, jedoch ist dann nach Beschreibung und Patentansprüchen der Europäischen Patentanmeldung 0 028 723 die Umsetzung in umständlicher Weise in mehreren Stufen durchzuführen: der Druck wird in mehreren Stufen bis auf 5 mbar zu senken und die Temperatur gleichzeitig in mehreren

- 2 -

Stufen auf schließlich 120 °C erhöht.

Es bestand daher die Aufgabe, N,N'-Diformylhydrazin aus Hydrazinhydrat unter Erhöhung des Umsatzes, Verbesserung der Ausbeute und Verminderung der Nebenproduktbildung auf technisch einfach auszuführende Weise herzustellen.

Gegenstand der Erfindung ist daher, ein Verfahren zur Herstellung von N,N'-Diformylhydrazin durch Umsetzung von Hydrazinhydrat oder ggf. Monoformylhydrazin mit Ameisensäure oder Formamid im Molverhältnis von 1 : 2 bis 1 : 2,5 bei erhöhter Temperatur, dadurch gekennzeichnet, daß man die Umsetzung in Gegenwart eines zwischen 40 und 120°C siedenden inerten Lösungsmittels, das mit Wasser ein azeotropes Gemisch bildet, bei der jeweiligen Siedetemperatur durchführt und das im Reaktionsgemisch enthaltene und das während der Reaktion gebildete Wasser durch azeotrope Destillation während der Umsetzung aus dem Reaktionsgemisch entfernt.

Gemäß der Erfindung ist es daher möglich, durch azeotrope Destillation während der Reaktion das zugeführte und entstandene Wasser vollständig zu entfernen, wobei je nach Siedetemperatur der in Art eines Schleppmittels verwendeten inerten Lösungsmittel die Temperatur im Reaktionsgefäß verhältnismäßig niedrig, nämlich auf der zur Abtreibung des Azeotrops erforderlichen Temperatur gehalten wird. Die bisher besonders bei Ameisensäure mit 16 bis 24 Stunden sehr lange Reaktionszeit wird bei einfacher technischer Handhabung auf 1 bis 4 Stunden gesenkt. Es war nicht vorauszusehen, daß durch Anwendung der azeotropen Wasserentfernung gegenüber bekannten bei Normaldruck arbeitenden Verfahren, eine derartige Steigerung der Reaktionsgeschwindigkeit und der Ausbeute erreicht werden konnte. Vielmehr war anzunehmen, daß mit dem Wasser erhebliche Mengen der bei 100,5°C siedenden Ameisensäure oder Hydrazin mit dem Siedepunkt 113,5$^o$C bzw. Hydrazinhydrat mit dem Siedepunkt 118,5$^o$C vor einem ausreichenden Umsatz abdestillieren und somit die Ausbeute erniedrigen würden. Außerdem war zu befürchten, daß sich die für zahlreiche Lösungsmittel bekannten ternären Azeotrope mit Wasser und Ameisensäure bilden. Es war auch nicht vorauszusehen, daß trotz der durch die niedrig siedenden inerten Lösungsmittel herabgesetzten Reaktionstemperatur die Reaktionsgeschwindigkeit nicht vermindert, sondern vielmehr wesentlich erhöht und die Reaktionsdauer auf 1 bis 4 Stunden verkürzt werden konnte.

Als Ausgangsstoffe werden Hydrazinhydrat, dessen wässrige Lösung oder ggf. Monoformylhydrazin verwendet, wobei wenigstens 50 gew.-%-ige Lösungen verwendet werden sollen. Ameisensäure bzw. Formamid werden als reine Stoffe oder als wässerige Lösungen von zweckmäßig mindestens 50 Gew.-% verwendet. Soweit ggf. Monoformylhydrazin und entsprechend verminderte Menge Ameisensäure oder Formamid eingesetzt werden, kann dieses als Feststoff eingesetzt werden, der sich im Reaktionsgemisch schnell löst.

Als inerte Lösungsmittel eignen sich solche mit Siedepunkten zwischen 40 und 120$^o$C, welche mit Wasser Azeotrope bilden und daher zur Ausschleppung des Wassers bei der azeotropen Destillation dienen können.

Geeignet sind beispielsweise aliphatische, cycloaliphatische oder aromatische Kohlenwasserstoffe für die genannten Siedepunkte beispielsweise n-Hexan, Cyclohexan, Toluol und bei Vorkehrungen gegen Gesundheitsschäden auch Benzol.

Geeignete inerte Lösungsmittel sind weiterhin aliphatische Clorkohlenwasserstoffe, wie beispielsweise 1,2-Dichlorethan, und Dialkylether wie beispielsweise Diisopropylether.

Zur Durchführung des erfindungsgemäßen Verfahrens werden die Reaktionskomponenten und die inerten Lösungsmittel vorgelegt oder eine der Reaktionskomponenten mit dem Schleppmittel vermischt vorgelegt und die andere bevorzugt so zudosiert, daß ohne zusätzliche Heizung die Reaktionstemperatur annähernd erreicht wird.

Die azeotrope Wasserentfernung und die Reaktion erfolgt unter Wärmezufuhr in einer zur Wasserabscheidung üblichen Apparatur. Das N,N'-Diformylhydrazin wird während der Reaktion in fester Form im Reaktionsgefäß abgeschieden und nach der Reaktion durch Filtration von der flüssigen Phase getrennt. Das Produkt, das nach dem Waschen mit reinem inertem Lösungsmittel und Trocknen zwischen 157 und 159$^o$C schmilzt (Literatur 159 bis 160$^o$C), besitzt ohne zusätzliche Reinigung die für die Weiterverarbeitung erforderliche Reinheit. Die vom Produkt abgetrennte flüssige Phase, die außer dem Schleppmittel ggf. geringe Mengen an Ausgangsstoffen enthält, wird in den Prozeß zurückgeführt. Die Reinigung der flüssigen Phase braucht wegen des sehr geringen Gehalts an Nebenprodukten nur in großen Abständen, beispielsweise durch Destillation oder Waschen mit Wasser, zu erfolgen. Soweit Formamid als Einsatzstoff verwendet wird, wird das übergetriebene Ammoniak übergetrieben und von Azeotrop getrennt.

N,N'-Diformylhydrazin dient besonders als Zwischenprodukt zur Herstellung von 1,2,4-Triazol und dessen Derivate.

- 5 -

## Beispiel 1

In einem Kolben mit Rückflußkühler und Wasserabscheider wurden 170 ml Ameisensäure (98 bis 100 Gew-%) mit 400 ml Toluol vermischt und unter Rühren 100 ml Hydrazinhydrat (ca. 99 Gew-%) innerhalb von 12 Minuten zugegeben, wobei die Temperatur des Gemisches von 22 auf 75°C anstieg. Durch Erhitzen auf kräftigen Rückfluß wurden in 150 Minuten unter Anstieg der Sumpftemperatur bis 106°C 129 g wässeriges Destillat abgetrennt. Anschließend wurden 100 ml Toluol abdestilliert, das zum Waschen des im Reaktionskolben abgeschiedenen und vom restlichen Toluol abgefilterten Kristallinen Produktes verwendet wurde. Nach dem Trocknen bei 100°C wurden 166 g farbloses N,N'-Diformylhydrazin vom Schmelzpunkt 157°C erhalten, entsprechend einer Ausbeute von 93% d.Th., bezogen auf eingesetztes Hydrazinhydrat.
Das wässerige Destillat enthält 10,2 g Ameisensäure.

## Beispiel 2

Zum Gemisch aus 85 ml (ca. 2,2 mol) Ameisensäure und 400 ml Diisopropylether wurden 50 ml (ca. 1 mol) Hydrazinhydrat in 10 Minuten gegeben, wobei sich das Gemisch auf 63°C erwärmte. Beim nachfolgenden Erhitzen destillierten bei einer Sumpftemperatur von 64 - 68°C in 190 Minuten 56 g Wasser mit einem Ameisensäuregehalt von 0,12% ab. Danach wurden 100 ml Diisopropylether abdestilliert, mit dem das abgeschiedene und abgetrennte N,N'-Diformylhydrazin gewaschen wurde. Ausbeute nach dem Trocknen: (98,8 % d.Th.), Schmelzpunkt: 158°C.

## Beispiel 3

Beispiel 2 wird wiederholt, jedoch unter Verwendung von 400 ml Cyclohexan als inertes Lösungsmittel und Schlepp-

- 6 -

mittel. Bei einer Sumpftemperatur von 70 - 76$^0$C wurden in 200 Minuten 61 ml wässeriges Destillat abgeschieden (Ameisensäuregehalt: 7,2%). Ausbeute an getrocknetem N,N-Diformylhydrazin: 87,2 g (97,7%), Schmelzpunkt: 157$^0$C.

Beispiel 4:

In 200 ml Diisopropylether wurden 60 g (ca. 1 mol) Monoformylhydrazin und 47 g (ca. 1 mol) Ameisensäure eingerührt, wobei die Temperatur nur wenig anstieg. Während des Erhitzens wurden bei einer Sumpftemperatur von 63 - 66$^0$C in 170 min 17 ml Wasser abgetrennt, das 0,3% Ameisensäure enthielt. Das mit 50 ml Diisopropylether gewaschene und bei 80$^0$C im Vakuum getrocknete N,N'-Diformylhydrazin schmolz bei 158$^0$C. Ausbeute: 86,8 g (98,6% d.Th.).

Beispiel 5:

In 500 ml 1,2-Dichlorethan werden 203,3 g (4,45 mol) Ameisensäure gelöst. In dieser Mischung werden unter Rühren 178,2 g wässerige 78 gew.%ige Hydrazinhydratlösung (2,0 mol) zugetropft. Unter gutem Rühren wird bis zur Siedetemperatur erhitzt und in 3,5 Stunden das Wasser als 152 g wässeriges Destillat mit 12,2% Ameisensäure-Gehalt abdestilliert. Anschließend werden 100 ml Lösungsmittel entfernt. Das im Reaktionskolben kristallin abgeschiedene Produkt wird abfiltriert und mit dem destillierten Lösungsmittel gewaschen und getrocknet, 169.4 (96.3% d.Th.) N,N'-Diformylhydrazin, Schmelzpunkt 157$^0$C.

Beispiel 6:

100 g Formamid (ca. 99 Gew.-%), werden mit 400 ml Toluol und 50 ml Hydrazinhydrat (99 Gew.-%), ca. 1 mol) gemischt und unter Rühren am Wasserabscheider erhitzt, wobei inner-

- 7 -

halb von 2 1/2 Stunden 23 ml $NH_3$-haltiges Wasser (entsprechend 19 g $H_2O$) abgeschieden werden. Anschließend werden
noch 100 ml Touol abdestilliert, mit dem das aus dem Reaktionsgemisch abfiltrierte kristalline Produkt gewaschen
wird. Nach 3 Stunden Trocknen bei 80°C erhält man 84,8 g farblose Kristalle von Schmp. 157°C, entsprechend einer Ausbeute
von 96,2 % d.Th., bezogen auf Hydrazinhydrat.

Troisdorf, 10. Dez. 1982
4211 Dr. La./cp OZ:83002

Patenansprüche

1. Verfahren zur Herstellung von N,N'-Diformylhydrazin durch Umsetzung von Hydrazinhydrat oder ggf. Monoformylhydrazin mit Ameisensäure oder Formamid im Molverhältnis von 1 : 2 bis 1 : 2,5 bei erhöhter Temperatur, dadurch gekennzeichnet, daß man die Umsetzung in Gegenwart eines zwischen 40 und 120°C siedenden inerten Lösungsmittels, das mit Wasser ein azeotropes Gemisch bildet, bei der jeweiligen Siedetemperatur durchführt und das im Reaktionsgemisch enthaltene und das während der Reaktion gebildete Wasser durch azeotrope Destillation während der Umsetzung aus dem Reaktionsgemisch entfernt.

2. Verfahren nach Anspruch 1, dadurch gekennzeichnet, daß als inerte, mit Wasser azeotrope Gemische bildende Lösungsmittel, aliphatische, cycloaliphatische oder aromatische Kohlenwasserstoffe mit Siedepunkten zwischen 40 und 120°C verwendet werden.

3. Verfahren nach Anspruch 1, dadurch gekennzeichnet, daß als inerte, mit Wasser azeotrope Gemische bildende Lösungsmittel, Halogenkohlenwasserstoffe mit Siedepunkten zwischen 40 und 120 °C verwendet werden.

4. Verfahren nach Anspruch 1, dadurch gekennzeichnet, daß als inerte, mit Wasser azeotrope Gemische bildende Lösungsmittel, Dialkylether mit Siedepunkten zwischen 40 und 120 °C verwendet werden.

5. Verfahren nach einem der vorangehenden Patentansprüche, dadurch gekennzeichnet, daß die Ausgangsstoffe als wässerige, vorzugsweise mindestens 50%ige Lösungen eingesetzt werden.

Europäisches
Patentamt

# EUROPÄISCHER RECHERCHENBERICHT

EP 83 11 2289

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | DE-A-2 917 087 (WERNER)<br>* Ansprüche; Seiten 3,4; Beispiele 13,14 * | 1-5 | C 07 C 109/087 |
| Y | US-A-3 023 241 (TWELVES)<br>* Beispiel 3 * | 1-5 | |

|  |  |
|---|---|
| | RECHERCHIERTE SACHGEBIETE (Int. Cl. 3) |
| | C 07 C 109/00 |

Der vorliegende Recherchenbericht wurde für alle Patentansprüche erstellt.

| Recherchenort | Abschlußdatum der Recherche | Prüfer |
|---|---|---|
| DEN HAAG | 27-07-1984 | PAUWELS G.R.A. |